# FASCICULE DE BREVET EUROPEEN

(11) **EP 1 807 516 B1**
(45) Date de publication et mention de la délivrance du brevet: **02.03.2011**
(21) Numéro de dépôt: 05809287.5
(22) Date de dépôt: 12.10.2005
(51) Int. Cl.: C12N 15/29

(54) **PROMOTEURS VEGETAUX ET UTILISATIONS**
PFLANZENPROMOTOREN UND VERWENDUNG DAVON
PLANT PROMOTERS AND USES THEREOF

(30) Priorité: 13.10.2004 FR 0410799
(43) Date de publication de la demande: 18.07.2007
(73) Titulaire: Philip Morris Products S.A., 2000 Neuchâtel (CH)
(72) Inventeur: TISSIER, Alain, F-84120 PERTUIS (FR); SALLAUD, Christophe, F-34070 MONTPELLIER (FR); RONTEIN, Denis, F-04100 MANOSQUE (FR)
(74) Mandataire: Gallois, Valérie
(86) Numéro de dépôt international: PCT/FR2005/002530
(87) Numéro de publication internationale: WO 2006/040479

(56) Documents cités:
- WO-A-2004/111183
- US-B2- 6 730 826
- WANG E. ET AL.: "Isolation and characterization of the CYP71D16 trichome-specific promoter from Nicotania tabacum L." JOURNAL OF EXPERIMENTAL BOTANY, vol. 53, no. 376, 2002, pages 1891-1897, XP002318244 cité dans la demande
- WAGNER G J: "SECRETING GLANDULAR TRICHOMES: MORE THAN JUST HAIRS" PLANT PHYSIOLOGY, AMERICAN SOCIETY OF PLANT PHYSIOLOGISTS, ROCKVILLE, MD, US, vol. 96, 1991, pages 675-679, XP002946021 ISSN: 0032-0889
- WANG ERMING ET AL: "Elucidation of the functions of genes central to diterpene metabolism in tobacco trichomes using posttranscriptional gene silencing." PLANTA (BERLIN), vol. 216, no. 4, février 2003 (2003-02), pages 686-691, XP002318258 ISSN: 0032-0935 cité dans la demande

### SEQUENCE LISTING

<110> LIBROPHYT
<120> Promoteurs végétaux et utilisations
<130> B0296WO
<150> FR 04 10799
   <151> 2004-10-13
<160> 23
<170> PatentIn version 3.1
<210> 1
   <211> 1068
   <212> DNA
   <213> Nicotiana sylvestris
<400> 1
   aatttatttt cataaaactt ctctaatttt tgaacaaaat cttatattga ttttttaatc     60
   aaagccaaaa tatttattta actatgaaaa ttttttaaca actaatttat tatggtaaat    120
   aatattgata tggtaacttc aagcacatga caaaaattat aactaactgc agaagtttac    180
   tgtctctctg aatcttgtgg ctatgtcatt ctatcataac aaatacttgt agctaatacg    240
   ccaacgatgt tctcgatttc atataatttg aattttaaaa tagcttttaa atttaatatt    300
   tatttcaaat cattattgtg actaacatgt tataaccgca gtaatatttg gagatgcaat    360
   acttatattt agctacaaaa ttttattgta tcataataag tttgtagcta ttaagttagt    420
   ttttgccaca aatttttata attgaagcaa aaatacctat tcaactacaa tattttgtat    480
   cgagtaatat tttgtgacta gaagattaat attattacag taatttcaga cgtgtggcaa    540
   aaactcataa ttatctacaa aatattgtcg tagcaataat tttttatatc tattaatcca    600
   attattgcta catgctttta taacttgagg caaaaatatc tatttagcta taacattttg    660
   ttagaagtaa tttttgtgac tataaagttg ttattgctac agtaatttca aatgcgtggc    720
   aaaaaaaata cgattagcta cgaaatttta ttgtagcaat aaatttgtag ctatttgggt    780
   aatattgcta cgacagttag caattatagc aaaaatgcta aatcagcttt gtcgatttaa    840
   ttttgtagct aattttttta tgaatttgta aatagctatg aaattttaat ttttgtggct    900
   attgttaggt attagccaca tatagctaag aatttgtagc tatatataca taatgttgta    960
   gtggcaaatt ctaacattgt aagcttggct gccttttttt ttttttggct acaaaactct   1020
   aaagtaaagg aactagaaaa ctcgtttggc gagagaaaga gagagatg                1068
<210> 2
   <211> 1060
   <212> DNA
   <213> Nicotiana sylvestris
<400> 2
   aatttatttt tgtaaaactt ctctaatttt tggacaaact cttatattga ttttttaatc      60
   aaagccaaaa tatttattta actatgaaaa aattttaaca actaatttat tatggtaaat     120
   aatattgata tggtaacttc aagcacatga caaaaattat aactaactgc agaagtttat     180
   tgtctctctg aatcttgtgg ctatatcata acaaatactt gtagctaata agccaacgat     240
   gttctcggtt tcatataatt tgaattttaa aatagttttt aaatttaata tttatttcaa     300
   atcattattg tggctaacat gttataatcg cagtaatatt tggagatgca atacttatat     360
   ttagctacaa aattttattg tatcagaata agtttgtagc tattaagtta gtttttgcca     420
   caaattttta taattgaagc aaaaatactt attcagctac agtattttgt atcgagtaat     480
   attttgtgac tagaagatta atattgctac agtaatttca gacgtgtggc aaaaactcat     540
   aattagctac aaaatattgt cgtagcaata attttttata tctattaatg caattattac     600
   tacatgcttt tataacttga ggcaaaaata tctaatagct ataacatttt gttagaagta     660
   atttttgtgg ctataaaatt ggtattgcta cagtaatttc aaatgcgtgg caaaaaaata     720
   cgattaacta cgaaatttta ttgtagcaat aactttgtag ctatttgggt aatattgcta     780
   cgacagttag caattatagc aaaaatgcta aatcagcttt gtcaatttaa ttttgtagct     840
   aattttttta tgaaattgta aatagctatg aaattttaat ttttgtggct attgttaggt     900
   attagccaca tatagctaag aatttgtagc tatatataca taatgttgta gtggcaaatt     960
   ctaacattgt acgcttggct gccctttttt ttttttttgg ctacaaaact ctaaagtaaa    1020
   ggaactagaa aactcgtttg gcgagagaaa gagagagatg                           1060
<210> 3
   <211> 1060
   <212> DNA
   <213> Nicotiana sylvestris
<400> 3
   aatttatttt cgtaaaattt ctctaatttg gacaaactct tatattgatt tttttaatca      60
   aagccaaaat atttatttaa ctatgaaaat tttttaacaa ctaatttatt atggtaaata 120
   atattgatat ggtaacttca agcacatgat aaaaattata actaactgca gaagtttact 180
   gtctctttga atcttgtggt tatatcattc tatcataaca aatacttgta gctaataagc 240
   caacgatgtt ctcggtttca tataatttga attttaaaat agtttttaaa tttaatattt 300
   atttcaaatt attattgtgg ctaacatgtt ataaccgcag taatatttgg agatgcaata 360
   cttatattta gcttgaaaat tttattgtat cagaacaagt ttgtagctat taagttagtt 420
   tttgccacaa atttttataa ttgaagcaaa aatacctatt cagctacagt attttgtatc 480
   gagtaatatt ttgtgactag aagattaata ttgctacagt aatttcagac gtgtggcaaa 540
   aactcataat tagctacaaa atattgtcgt agcaataatt ttttatatct attaatccaa 600
   ttattgctac atgcttttat aacttgaggc aaaaatatct atttagctat aacattttat 660
   taaaagtaat ttttgtggct ataaagttgt tattgctaca gtaatttcaa atgcgtggca 720
   aaaaaaatac gattagctac gaaattttat tgtagcaata aatttgtagc tatttgggta 780
   atattgctac gacagttagc aattatagca aaaatgctaa attagctttg ttaatttaat 840
   tttgtagcta aactttttta tgaaatttta atttttgtgg ctattgatag gtattagcta 900
   caattttcat atatgtagct aagaatttgt agctatatat acataatgtt gtagtggcaa 960
   attctaacat tgtacgcttg gctgcccttt ttttttggct acaaaactct aaagtaaagg 1020
   aactagaaaa ctcgtttggc gagagaaaga gagagagatg 1060
<210> 4
   <211> 1087
   <212> DNA
   <213> Nicotiana sylvestris
<400> 4
   aatttatttt cgtaaaattt ctctaatttt tggacaaact cttatattgg ttttttaatc 60
   aaagccaaaa tatttattta actatgaaat tttgttgaac aactaattta ttatggtaaa 120
   taatattgat atggtaactt caagcacatg acaaaaatta taactaactg cagaagttta 180
   ctgtctctct gaatcttgtg gctatatcat tctatataac aaatacttgt agctaataag 240
   ccaacgatgt tctcggtttc atataatttg aattttaaaa tagtttttaa atttaatatt 300
   tatttcaaat cattattgtg gctagcatgt tataaccgca gtaatatttg gagatgcaat 360
   acttatattt agctacaaaa ttttattgta tcagaataag tttgtaacta ttaagttagt 420
   ttttgccaca aatttttata attgaagcaa aaatacctat tcagctacga tattttgtat 480
   cgagtaatat tttgtgacta gaagattaat attgctagag taatttcaga cgtgtggcaa 540
   aaactcataa ttagctacaa aatattgtcg tagcaataat tgtttatatc tattaatcca 600
   attattgcta tatgctttta taacttgagg caaaaatatt tatttagcta taacattttg 660
   ttagaagtaa tttttgtggc tataaagttg ttattgctac ggtaatttca aatgcgtggc 720
   aaacaaatac gattagctac gaaattttat tgtagcaata aatttgtagc tatttgggta 780
   atattgctac gacagttagc aattatagca aaaatgctaa attagctttg tcaatttaat 840
   tttttagcta aattttttta taaaattgta aatagccatg aaattttaat ttttgtggct 900
   attgttaggt attagccaca attttcatat atgtatctaa gaatttgtag ctatatatac 960
   ataatgttgt agtggcaaat tctaacattg taagcttagc tgcccttttt tttttttttt 1020
   tttggctaca aaactctaaa gtaaaggaac tagaaaactc gtttggcgag agaaagaggg 1080
   atccatg 1087
<210> 5
   <211> 2896
   <212> DNA
   <213> Artificial sequence
<220>
   <223> fusion du promoteur TPS02b avec le gène uidA
<400> 5
   aagcttaatt tatttttgta aaacttctct aatttttgga caaactctta tattgatttt     60
   ttaatcaaag ccaaaatatt tatttaacta tgaaaaaatt ttaacaacta atttattatg    120
   gtaaataata ttgatatggt aacttcaagc acatgacaaa aattataact aactgcagaa    180
   gtttattgtc tctctgaatc ttgtggctat atcataacaa atacttgtag ctaataagcc    240
   aacgatgttc tcggtttcat ataatttgag ttttaaaata gtttttaaat ttaatattta    300
   tttcaaatca ttattgtggc taacatgtta taatcgcagt aatatttgga ggtgcaatac    360
   ttatatttag ctacaaaatt ttattgtatc agaataagtt tgtagccatt aagttagttt    420
   tcgccacaaa tttttataat tgaagcaaaa atacttattc agctacagca ttttgtatcg    480
   agtaatattt cgtgactaga agattaatat tgctacagta atttcagacg tgtggcaaaa    540
   actcataatt agctacaaaa tattgtcgta gcaataattt tttatatcta ttaatgcaat    600
   tattactaca tgcttttata acttgaggca aaaatatcta atagctataa cattttgtta    660
   gaagtaattt ttgtggctat aaaattggta ttgctacagt aatttcaaat gcgtggcaaa    720
   aaaatacgat taactacgaa attttattgt agcaataact ttgtagctat ttgggtaata    780
   ttgctacgac agttagcaat tatagcaaaa atgctaaatc agctttgtca atctaatttt    840
   gtagctaatt tttttatgaa attgtaaata gctatgaaat tttaattttt gtggctattg    900
   ttaggtatta gccacatata gctaagaatt tgtagctata tatacataat gttgtagtgg    960
   caaattctaa cattgtacgc ttggctgccc tttttttttt ttttggctac aaaactctaa   1020
   agtaaaggaa ctagaaaact cgtttggcga gagaaagagg gatcccccgg gtggtcagtc  1080
   ccttatgtta cgtcctgtag aaaccccaac ccgtgaaatc aaaaaactcg acggcctgtg   1140
   ggcattcagt ctggatcgcg aaaactgtgg aattgatcag cgttggtggg aaagcgcgtt 1200
   acaagaaagc cgggcaattg ctgtgccagg cagttttaac gatcagttcg ccgatgcaga 1260
   tattcgtaat tatgcgggca acgtctggta tcagcgcgaa gtctttatac cgaaaggttg 1320
   ggcaggccag cgtatcgtgc tgcgtttcga tgcggtcact cattacggca aagtgtgggt 1380
   caataatcag gaagtgatgg agcatcaggg cggctatacg ccatttgaag ccgatgtcac 1440
   gccgtatgtt attgccggga aaagtgtacg tatcaccgtt tgtgtgaaca acgaactgaa 1500
   ctggcagact atcccgccgg gaatggtgat taccgacgaa aacggcaaga aaaagcagtc 1560
   ttacttccat gatttcttta actatgccgg aatccatcgc agcgtaatgc tctacaccac 1620
   gccgaacacc tgggtggacg atatcaccgt ggtgacgcat gtcgcgcaag actgtaacca 1680
   cgcgtctgtt gactggcagg tggtggccaa tggtgatgtc agcgttgaac tgcgtgatgc 1740
   ggatcaacag gtggttgcaa ctggacaagg cactagcggg actttgcaag tggtgaatcc 1800
   gcacctctgg caaccgggtg aaggttatct ctatgaactg tgcgtcacag ccaaaagcca 1860
   gacagagtgt gatatctacc cgcttcgcgt cggcatccgg tcagtggcag tgaagggcga 1920
   acagttcctg attaaccaca aaccgttcta ctttactggc tttggtcgtc atgaagatgc 1980
   ggacttgcgt ggcaaaggat tcgataacgt gctgatggtg cacgaccacg cattaatgga 2040
   ctggattggg gccaactcct accgtacctc gcattaccct tacgctgaag agatgctcga 2100
   ctgggcagat gaacatggca tcgtggtgat tgatgaaact gctgctgtcg gctttaacct 2160
   ctctttaggc attggtttcg aagcgggcaa caagccgaaa gaactgtaca gcgaagaggc 2220
   agtcaacggg gaaactcagc aagcgcactt acaggcgatt aaagagctga tagcgcgtga 2280
   caaaaaccac ccaagcgtgg tgatgtggag tattgccaac gaaccggata cccgtccgca 2340
   aggtgcacgg gaatatttcg cgccactggc ggaagcaacg cgtaaactcg acccgacgcg 2400
   tccgatcacc tgcgtcaatg taatgttctg cgacgctcac accgatacca tcagcgatct 2460
   ctttgatgtg ctgtgcctga accgttatta cggatggtat gtccaaagcg gcgatttgga 2520
   aacggcagag aaggtactgg aaaaagaact tctggcctgg caggagaaac tgcatcagcc 2580
   gattatcatc accgaatacg gcgtggatac gttagccggg ctgcactcaa tgtacaccga 2640
   catgtggagt gaagagtatc agtgtgcatg gctggatatg tatcaccgcg tctttgatcg 2700
   cgtcagcgcc gtcgtcggtg aacaggtatg gaatttcgcc gattttgcga cctcgcaagg 2760
   catattgcgc gttggcggta acaagaaagg gatcttcact cgcgaccgca aaccgaagtc 2820
   ggcggctttt ctgctgcaaa aacgctggac tggcatgaac ttcggtgaaa aaccgcagca 2880
   gggaggcaaa caatga 2896
<210> 6
   <211> 12
   <212> DNA
   <213> Artificial sequence
<220>
   <223> Motif 1
<400> 6
   kktcgtwgca wt 12
<210> 7
   <211> 10
   <212> DNA
   <213> Artificial sequence
<220>
   <223> Motif 2
<400> 7
   agtaatwtyw 10
<210> 8
   <211> 10
   <212> DNA
   <213> Artificial sequence
<220>
   <223> Motif 3
<400> 8
   ttgtagcwaw 10
<210> 9
   <211> 171
   <212> DNA
   <213> Nicotiana sylvestris
<220>
   <221> CDS
   <222> (1)..(171)
   <223>
<400> 9
   atg agt caa tca att tct cca tta atg ttt tct cac ttt gca aaa ttt        48
   Met Ser Gln Ser Ile Ser Pro Leu Met Phe Ser His Phe Ala Lys Phe
   1               5                   10                  15
   cag tcg aat att tgg aga tgc aat act tct caa ctc aga gtt ata cac        96
   Gln Ser Asn Ile Trp Arg Cys Asn Thr Ser Gln Leu Arg Val Ile His
               20                  25                  30
   tca tca tat gcc tct ttt gga ggg aga aga aaa gag aga gta aga aga       144
   Ser Ser Tyr Ala Ser Phe Gly Gly Arg Arg Lys Glu Arg Val Arg Arg
           35                  40                  45
   atg aat cga gca atg gat ctt tct tca                                   171
   Met Asn Arg Ala Met Asp Leu Ser Ser
       50                  55
<210> 10
   <211> 57
   <212> PRT
   <213> Nicotiana sylvestris
<400> 10
   Met Ser Gln Ser Ile Ser Pro Leu Met Phe Ser His Phe Ala Lys Phe
   1               5                   10                  15
   Gln Ser Asn Ile Trp Arg Cys Asn Thr Ser Gln Leu Arg Val Ile His
               20                  25                  30
   Ser Ser Tyr Ala Ser Phe Gly Gly Arg Arg Lys Glu Arg Val Arg Arg
           35                  40                  45
   Met Asn Arg Ala Met Asp Leu Ser Ser
       50                  55
<210> 11
   <211> 29
   <212> DNA
   <213> Artificial sequence
<220>
   <223> amorce
<400> 11
   ctaagcttaa tttatttttg taaaacttc 29
<210> 12
   <211> 30
   <212> DNA
   <213> artificial sequence
<220>
   <223> amorce
<400> 12
   atggatccct ctttctctcg ccaaacgagt 30
<210> 13
   <211> 29
   <212> DNA
   <213> Artificial sequence
<220>
   <223> Amorce
<400> 13
   tggatcctga agaaagatcc attgctcga 29
<210> 14
   <211> 44
   <212> DNA
   <213> artificial sequence
<220>
   <223> amorce
<400> 14
   ctaatacgac tcactatagg gctcgagcgg ccgcccgggg aggt 44
<210> 15
   <211> 8
   <212> DNA
   <213> artificial sequence
<220>
   <223> amorce
<400> 15
   acctcccc 8
<210> 16
   <211> 27
   <212> DNA
   <213> artificial sequence
<220>
   <223> amorce
<400> 16
   ggatcctaat acgactcact atagggc 27
<210> 17
   <211> 26
   <212> DNA
   <213> artificial sequence
<220>
   <223> amorce
<400> 17
   agaggcatat gatgagtgta taactc 26
<210> 18
   <211> 18
   <212> DNA
   <213> artificial sequence
<220>
   <223> amorce
<400> 18
   ctatagggct cgagcggc 18
<210> 19
   <211> 25
   <212> DNA
   <213> artificial sequence
<220>
   <223> amorce
<400> 19
   acctccaaat attcgactga aattt 25
<210> 20
   <211> 25
   <212> DNA
   <213> artificial sequence
<220>
   <223> amorce
<400> 20
   gtcatgtgct tgaagttacc atatc 25
<210> 21
   <211> 26
   <212> DNA
   <213> artificial sequence
<220>
   <223> amorce
<400> 21
   gttaccatat caatattatt taccat 26
<210> 22
   <211> 1796
   <212> DNA
   <213> Nicotiana sylvestris
<400> 22
   gaattcacta gtgatttgag ctcaaagagg tgaaacctaa tctagtatgc aaaccatgtt      60
   aaattctcaa ttgttttgat agataatgag ttttctgata attaataaat tattagataa     120
   ttaaaggacc aaatttatat gacttttgtt ttttatcatc ttgatcatat atacaatgta     180
   atggatacaa gcttatagtt gtataaattc tatataatta gttattcata cattaattag     240
   atatattcaa ttgttcttta taaatataat tcaaacctga aagcaatact tattttgtaa     300
   gaattgcaat attgttattt tgttatggac ttaaatatta accatgttat aatcttaagt     360
   ttatattatt agaaaaactt agtttttgaa agactaatat gaacattagt acttatttca     420
   aaaataagcg cttagatata tgaaattact ttaagtactt atttaaaata attaagtacc     480
   acacatacat acatatctct acaaactgtt aaagttttct atatgagtac ttattttaaa     540
   ataagagcat aaatataata aattatgtta aattcttatt taaaataata aaggaccaaa     600
   catgcataaa ataaagtatg agcttaataa gtcaagaagc taattgataa gcattgatgc     660
   caaatgcact tactaactgt tctatattgt aggaaaaatc taacttttat attaaaaatt     720
   tattttcata aaacttccct aatttttgaa caaaatctta tattgatttt ttaatcaaag    780
   ccaaaatatt tatttaacta tgaaaatttt ttaacaacta atttattatg gtaaataata    840
   ttgatatggt aactttcagc acatgacaaa aattataact aactgcagaa gtttactgtc    900
   tctctgaatc ttgtggctat gtcattctat cataacaaat acttgtagct aatacgccaa    960
   cgatgttctc gatttcatat aatttgaatt ttaaaatagc ttttaaattt aatatttatt   1020
   tcaaatcatt attgtgacta acatgttata accgcagtaa tatttggaga tgcaatactt   1080
   atatttagct acaaaatttt attgtatcat aataagtttg tagctattaa gttagttttt   1140
   gccacaaatt tttataattg aagcaaaaat acctattcaa ctacaatatt ttgtatcgag   1200
   taatattttg tgactagaag attaatatta ttacagtaat ttctgacgtg tggcaaaaac   1260
   tcataattat ctacaaaata ttgtcgtagc aataattttt tatatctatt aatccaatta   1320
   ttgctacatg cttttataac ttgaggcaaa aatatctatt tagctataac attttgttag   1380
   aagtaatttt tgtgactata aagttgttat tgctacagta atttcaaatg cgtggcaaaa   1440
   aaaatacgat tagctacgaa attttattgt agcaataaat ttgtagctat ttgggtaata   1500
   ttgctacgac agttagcaat tatagcaaaa atgctaaatc agctttgtcg atttaatttt   1560
   gtagctaatt tttttatgaa tttgtaaata gctatgaaat tttaattttt gtggctattg   1620
   ttaggtatta gccacatata gctaagaatt tgtagctata tatacataat gttgtagtgg   1680
   caaattctaa cattgtaagc ttggctgcct tttttttttt ttgggctaca aaactctaaa   1740
   gtaaaggaac tagaaaactc gtttggcgag agaaagaggg atccataatc gaattc       1796
<210> 23
   <211> 36
   <212> DNA
   <213> artificial sequence
<220>
   <223> amorce
<400> 23
   tgagctcaaa gaggtgaaac ctaatctagt atgcaa 36

## Description

La présente demande concerne des outils, méthodes et compositions pour la modification de plantes et/ou l'expression de protéines dans des plantes. Elle concerne notamment des promoteurs transcriptionnels permettant une expression spécifique dans les trichomes, des constructions contenant ces promoteurs, et leurs utilisations pour modifier génétiquement des cellules, graines ou plantes. L'invention concerne également des procédés de production de plantes transgéniques exprimant des protéines ou métabolites d'intérêt. L'invention est applicable de manière générale à toute plante possédant des trichomes glanduleux, et à l'expression de toute protéine d'intérêt industriel, notamment thérapeutique ou phytosanitaire.

### INTRODUCTION

Les feuilles de certaines plantes supérieures (Angiospermes) possèdent à leur surface des organes dénommés trichomes qui, en fonction de leur anatomie, sont principalement divisés en deux types : les trichomes non sécréteurs d'une part et les trichomes sécréteurs d'autre part. Les trichomes glanduleux sécréteurs ont pour principale fonction de sécréter à la surface de la feuille des huiles ou essences composées de quelques molécules majoritaires.

Chez le tabac, les trichomes glanduleux sécréteurs sont composés d'un pied constitué d'une file de quelques cellules (3-5) et d'une tête rassemblant un nombre variable (2-20) de cellules sécrétrices. Ces cellules sécrètent majoritairement des esters de sucrose et des diterpènes. Ces derniers peuvent représenter jusqu'à 60% de l'exsudat et 10% du poids sec de la plante adulte (Wagner *et al.,* 2004). Chez les espèces cultivées de tabac *(Nicotiana tabacum),* les diterpènes produits appartiennent à deux familles distinctes, les labdanes et les cembranes. Les taux de production relatifs des deux familles sont dépendants du cultivar avec cependant une production de cembranes qui est généralement 4 à 10 fois plus forte que la production de labdanes. Chez *N. sylvestris,* l'un des progéniteurs supposés de *N. tabacum,* les labdanes sont absents et un composé appartenant à la famille des cembranes, le cembratriène-diol (CBT-diol), représente à lui seul plus de 60% des terpènes produits par la plante.

Les étapes conduisant à la biosynthèse du CBT-diol chez le tabac sont partiellement connues et peuvent se décomposer en deux parties distinctes:
- la biosynthèse du précurseur universel de tous les diterpènes, le géranylgéranyl pyrophosphate (GGPP), produit par la voie dite de « Rohmer » (Rohmer *et al.,* 1996), s'effectue dans le chloroplaste.
- La biosynthèse du CBT-diol s'effectue à partir du GGPP. Il a été proposé par Wang et Wagner (2003) le schéma de biosynthèse suivant :
   GGPP → CBT-ol → CBT-diol.

La première étape de cyclisation serait réalisée par une enzyme appartenant à une large famille d'enzymes connues sous le nom de terpène synthases (Bohlmann *et al.,* 1998). La diterpène synthase de tabac utiliserait le GGPP comme substrat pour former du CBT-ol. La seconde étape permettant de produire du CBT-diol à partir du CBT-ol est une étape d'hydroxylation catalysée par une enzyme appartenant à la famille des cytochromes P450. L'équipe du Professeur G. Wagner (University of Kentucky) a identifié, par PCR soustractive, deux gènes candidats *de N. tabacum* pour chacune de ces étapes :
- une séquence présentant une forte similarité avec des séquences codant pour des terpène-synthases (CYC-2 ; N° Genbank AF401234. NID : AY495694).
- une séquence codant pour une enzyme de type cytochrome P450 (CYP71D16, NID : AF166332) (Wang *et al.,* 2001, Wang & Wagner 2003).

Des expériences d'extinction de l'expression de ces gènes par co-suppression et RNAi chez le *N. tabacum* ont montré (i) une diminution de CBT-diol et de CBT-ol corrélée avec une diminution de l'expression du gène CYC-2, et (ii) une augmentation de l'accumulation de CBT-ol et une diminution de CBT-diol en corrélation avec une diminution de l'expression du gène CYC71D16 dans les trichomes. Ces travaux ont suggéré que (i) le gène CYC-2 code pour la CBT-ol cyclase responsable de la synthèse de CBT-ol et (ii) le gène CYP71D16 code pour une CBT-ol hydroxylase responsable de la synthèse de CBT-diol à partir du CBT-ol. Par ailleurs, une séquence génomique d'un gène très proche du mRNA CYC-2 a été récemment déposée dans les bases de données (CYC-1, NID : AY049090), ce qui suggère l'existence non pas d'un seul mais de plusieurs gènes de CBT-ol cyclases.

L'expression de protéines dans les plantes et/ou la modification génétique des plantes pour leur conférer des propriétés particulières, représentent un attrait majeur, à la fois dans le domaine phytosanitaire et pharmaceutique. La mise à disposition d'outils capables de contrôler ou réguler l'expression de gènes dans les plantes constitue donc un élément clé dans l'exploitation et le développement de ces systèmes. Le développement de promoteurs ou autres séquences régulatrices spécifiques de tissus, notamment des trichomes sécréteurs, constituerait à cet égard un atout très important.

Dans ce contexte, l'équipe de G. Wagner a montré qu'une séquence régulatrice de 1852 pb, située en amont de l'ATG du gène CYP71D16, permet de diriger l'expression du gène rapporteur *uidA* spécifiquement dans les cellules sécrétrices de trichomes de tabac (demande US2003/0100050 A1, Wagner et al., 2003). D'autre part, plusieurs séquences promotrices extraites de différentes espèces ont été identifiées comme étant capables de diriger l'expression d'un gène hétérologue dans les trichomes de tabac (Tableau 1).

Parmi ces promoteurs, celui du gène *LTP3,* codant pour une protéine du coton impliquée dans le transfert des lipides (LTP), s'exprime spécifiquement dans les cellules de fibres de coton. La séquence régulatrice du gène (1548 pb) a été étudiée chez le tabac. Cette séquence dirige de manière spécifique l'expression du gène *uidA* dans les trichomes de la feuille. La séquence de 315 pb localisée entre les positions -614 et -300 en amont de l'ATG serait responsable de la spécificité du promoteur. Le promoteur du gène *LTP6* permettrait également une expression spécifique des trichomes de coton. Sur la base des publications, il semblerait néanmoins que l'expression se localise dans les cellules de la base du trichome, et non dans les cellules sécrétrices. En outre, lorsque ces promoteurs sont introduits dans le tabac, l'expression n'est plus spécifique, avec notamment un signal dans les cellules épidermiques (cf Tableau 1).

Il existe donc aujourd'hui un besoin important de promoteurs ou séquences régulatrices de l'expression, adaptées à l'expression de gènes dans des plantes, et permettant notamment une expression forte et spécifique de tissus, en particulier des trichomes sécréteurs.

### RESUME DE L'INVENTION

La présente demande décrit l'identification et la caractérisation de séquences d'acide nucléique régulatrices d'origine végétale, qui permettent de diriger de manière spécifique l'expression d'un gène d'intérêt dans les cellules sécrétrices des trichomes de plante. Ces promoteurs sont aisément manipulables, de taille raisonnable, modulables, adaptables à l'expression de gènes hétérologues, et capables de causer une expression spécifiquement dans certains tissus végétaux. La présente demande décrit ainsi des promoteurs, cassettes d'expression et vecteurs utilisables pour la modification de plantes et l'expression de produits d'intérêt dans des cellules ou tissus végétaux.

Un premier objet de l'invention réside plus particulièrement dans un acide nucléique ayant une activité de promoteur transcriptionnel fonctionnel spécifique des cellules sécrétrices dans les trichomes glanduleux, caractérisé en ce qu'il comprend une séquence sélectionnée parmi la SEQ ID NO: 2, un fragment de celles-ci d'au moins 100 bases consécutives ou un variant fonctionnel de celles-ci présentant au moins 80% d'identité avec celle-ci ou ledit fragment. Plus particulièrement, l'acide nucléique comprend une séquence sélectionnée parmi les SEQ ID NOS: 1, 2, 3, 4 et 22, et un fragment de celles-ci d'au moins 100 bases consécutives , et est spécifique des cellules sécrétrices des trichomes glanduleux. La demande décrit un peptide de transit dans le chloroplaste de cellules végétales, comprenant la séquence MSQSISPLMFSHFAKFQSNIWRCNTSQLRVIHSSYASFGGRRKERVRRMNRAM DLSS (SEQ ID NO : 10) ou un fragment fonctionnel de celle-ci, ainsi que dans un acide nucléique codant un tel peptide.

L'invention concerne également toute cassette d'expression recombinante comprenant un gène d'intérêt lié de manière opérationnelle à un acide nucléique tel que défini ci-dessus ; tout vecteur d'expression comprenant un acide nucléique ou une cassette tels que définis ci-dessus ; et toute cellule modifiée comprenant une cassette ou un vecteur tels que définis ci-dessus. De préférence, la cellule est une cellule de plante, notamment de la famille des Solanacées, Astéracées, Cannabacées ou Lamiacées.

Un autre objet de l'invention concerne un procédé de production d'une protéine dans les trichomes d'une plante, comprenant l'introduction dans ladite plante d'une cassette ou d'un vecteur tels que définis ci-dessus, comprenant un gène codant ladite protéine.

Un autre objet de l'invention concerne un procédé de production d'une protéine dans les trichomes d'une plante, comprenant l'introduction dans une cellule ou graine de plante d'une cassette ou d'un vecteur tels que définis ci-dessus, comprenant un gène codant ladite protéine, et la reconstitution d'une plante à partir de ladite cellule ou graine.

Un autre objet de l'invention concerne un procédé de production d'une plante exprimant une protéine recombinante, comprenant l'introduction dans une cellule ou graine de plante d'une cassette ou d'un vecteur tels que définis ci-dessus, comprenant un gène codant ladite protéine, et la reconstitution d'une plante à partir de ladite cellule ou graine. Avantageusement, ladite protéine est une enzyme dont l'activité dans les cellules sécrétrices conduit à une modification de la composition de l'exsudat.

Un autre avantage consiste en ce que la plante sécrète la protéine dans le trichome glanduleux, et la protéine est récupérée à partir de l'exsudat de la surface des feuilles.

Un autre objet de l'invention concerne toute plante ou graine comprenant une cassette d'expression ou un vecteur tels que définis ci-dessus.

L'invention vise également l'utilisation d'un acide nucléique tel que défini ci-dessus pour l'expression d'une protéine spécifiquement dans les trichomes glanduleux d'une plante. Elle concerne également des kits comprenant les acides nucléiques, vecteurs, cassettes et/ou cellules tels que décrits précédemment.

Comme il sera décrit en détails dans la suite du texte, la présente invention est applicable à l'expression de tout gène d'intérêt dans toute plante ou tissu végétal, de préférence des plantes supérieures comprenant des trichomes glanduleux, pour des applications variées, comme la production de produits d'intérêt pharmaceutique ou phytosanitaire, ou la production de plantes ayant des propriétés améliorées ou adaptées.

### DESCRIPTION DETAILLEE DE L'INVENTION

La présente demande décrit l'identification de promoteurs spécifiques des trichomes de plantes à partir de gènes de CBT-ol synthase de tabac. A partir de la séquence d'un ADNc codant pour une terpène synthase, i.e. CBT-ol cyclase (CYC-2 ; NID : AF401234), quatre gènes (notés NsTPS-02a, 02b, 03, et 04) possédant une forte similarité de séquence avec CYC-2 ont été identifiés à partir d'ADN génomique de l'espèce *Nicotiana Sylvestris,* en utilisant des techniques faisant appel à la PCR.

Une analyse de l'expression a montré que ces gènes (notamment NsTPS-02a, 02b, et 03) s'expriment dans les trichomes de tabac.

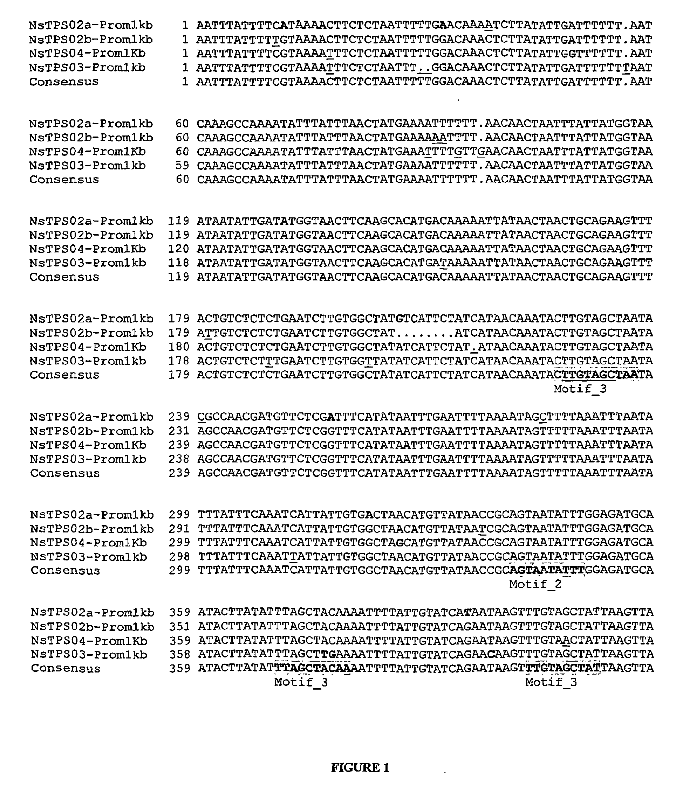

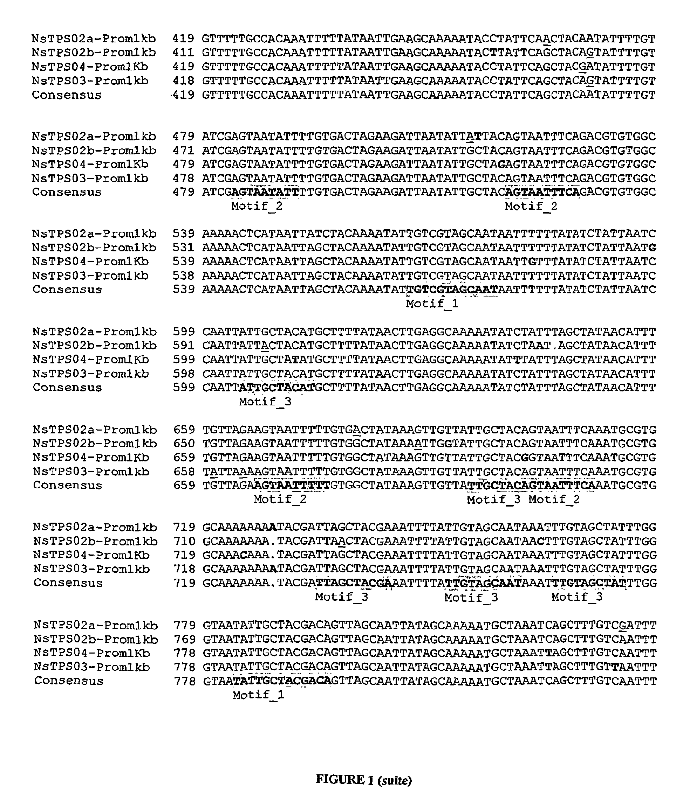

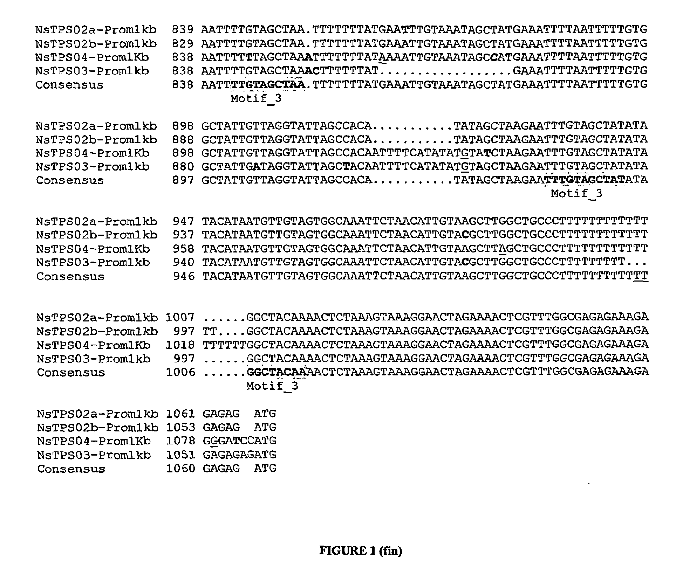

Les séquences promotrices, inconnues à ce jour, situées en amont de l'ATG de chacun de ces quatre gènes, ont été identifiées et caractérisées. La taille de chacune des séquences est d'environ 1 kb. Leurs séquences sont représentées dans les SEQ ID NO: 1 à 4. L'alignement des quatre séquences montre qu'elles possèdent plus de 93% d'identité entre elles (Figure 1, Tableau 2). En outre, cet alignement permet d'identifier des régions identiques, certainement impliquées dans la spécificité d'expression.

La construction de cassettes d'expression recombinantes utilisant ces promoteurs a permis de montrer, de manière inattendue, un profil d'expression restreint et spécifique des cellules des trichomes sécréteurs. Un tel profil était inattendu dans la mesure où les caractéristiques d'expression des terpène-synthases n'étaient nullement documentées dans l'art antérieur. D'ailleurs, les résultats obtenus par les demandeurs montrent même que le gène CYC-1 (NID : AY049090) dont la séquence génomique avait été déposée par G. Wagner, est peu exprimé dans les trichomes de *N. tabacum.*

La présente demande fournit donc des promoteurs nouveaux, particulièrement avantageux pour l'expression de protéines dans des plantes ou tissus végétaux, notamment pour l'expression spécifique dans les cellules des trichomes glanduleux. De tels promoteurs sont particulièrement avantageux puisqu'ils permettent la production de produits recombinants directement dans l'exsudat sécrété par les feuilles, permettant ainsi une récupération très aisée des produits recombinants, le cas échéant.

Un objet particulier de l'invention réside donc dans tout acide nucléique ayant une activité de promoteur transcriptionnel fonctionnel dans les trichomes glanduleux, caractérisé en ce qu'il comprend une séquence sélectionnée parmi les SEQ ID NOS: 1, 2, 3, 4 et 22, un fragment de celles-ci d'au moins 100 bases consécutives ou un variant fonctionnel de celles-ci (ou de leur brin complémentaire).

Dans un mode de mise en oeuvre particulier, l'invention concerne tout acide nucléique tel que défini ci-dessus, caractérisé en ce qu'il comprend une séquence sélectionnée parmi les SEQ ID NOS: 1, 2, 3, 4 et 22, un fragment de celles-ci d'au moins 100 bases consécutives ou une séquence présentant au moins 80% d'identité avec l'une d'entre-elles, et en ce qu'il possède une activité de promoteur transcriptionnel spécifique des trichomes glanduleux.

Dans un mode de réalisation spécifique de l'invention, l'acide nucléique comprend une séquence sélectionnée parmi les SEQ ID NOS: 1, 2, 3, 4 et 22, de préférence de la séquence SEQ ID NO: 2.

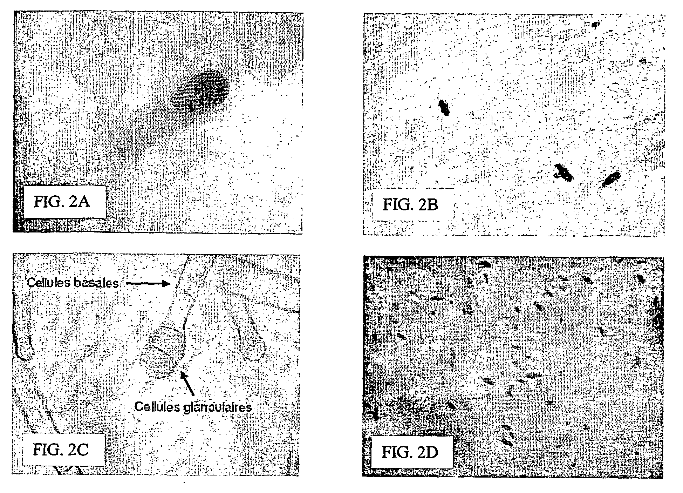

Comme illustré dans les exemples, la séquence de 1065 paires de bases du promoteur du gène TPS-02b (SEQ ID NO: 2) a été fusionnée en amont d'un gène rapporteur *uidA* (la séquence de la fusion du promoteur TPS02b avec le gène *uidA* est représentée dans SEQ ID NO: 5). Cette construction (cassette N°1) a été introduite dans un vecteur T-DNA (pBI121) possédant un gène de résistance pour la kanamycine sous la dépendance du promoteur de la nopaline synthase d'agrobacterium Tumefasciens (nos). Ce T-DNA a été introduit dans *N. sylvestris* par transformation génétique en utilisant la souche *d'Agrobacterium tumefaciens* LBA4404 (Hoekema *et al.,* 1993). L'expression du gène *uidA* dans plusieurs transformants a été détectée. Les résultats présentés sur la figure 2 montrent une expression dans les cellules sécrétrices, mais pas dans les cellules constituant le pied des trichomes. En outre, aucune expression du gène *uidA* n'a été trouvée dans les autres organes de la plante. Ces résultats démontrent que la séquence étudiée permet de diriger l'expression d'un gène hétérologue dans les cellules sécrétrices des trichomes glanduleux de manière très spécifique.

Au sens de l'invention, on entend par « acide nucléique » toute molécule à base d'ADN ou d'ARN. Il peut s'agir de molécules synthétiques ou semi-synthétiques, recombinantes, éventuellement amplifiées ou clonées dans des vecteurs, modifiées chimiquement ou comprenant des bases non-naturelles. Il s'agit typiquement de molécules d'ADN isolées, synthétisées par des techniques recombinantes bien connues en soi de l'homme du métier.

Au sens de l'invention, on entend par promoteur "spécifique" un promoteur principalement actif dans un tissu où groupe cellulaire donné. Il est entendu qu'une expression résiduelle, généralement plus faible, dans d'autres tissus ou cellules ne peut être entièrement exclue. Par exemple, l'expression résiduelle dans d'autres tissus ou cellules ne dépasse pas 30 % de l'expression observée dans les trichomes glanduleux, de préférence ne dépasse pas 20 %, 10% ou 5 %. Une caractéristique particulière de l'invention réside dans la capacité de construire des promoteurs spécifiques des cellules sécrétrices des trichomes glanduleux, permettant une modification de la composition des sécrétions foliaires de la plante, et notamment d'y exprimer des produits d'intérêt.

Un "fragment" d'un acide nucléique selon l'invention désigne avantageusement un fragment comprenant au moins 10 bases consécutives, plus généralement au moins 20, 30, 40, 50, 60, 70, 80, 90 ou 100. Des "fragments" d'un acide nucléique sont typiquement des fragments de 100, 200, 300, 400 ou 500 nucléotides consécutifs de la séquence ou plus. Des "fragments" d'un acide nucléique sont de préférence des fragments d'au moins 100, 200, 300, 400 ou 500 nucléotides consécutifs de la séquence. Les "fragments" d'un acide nucléique peuvent être utilisées seuls, ou combinés à d'autres régions transcriptionnelles, pour constituer des promoteurs chimériques ayant une spécificité pour les trichomes glanduleux. Un promoteur selon l'invention est typiquement une région de la séquence possédant une activité de promoteur.

La figure 1 montre que les promoteurs de l'invention possèdent un pourcentage d'identité très élevé entre eux. A cet égard, l'analyse de ces séquences a permis aux inventeurs d'identifier des parties de ces séquences comme des motifs conservés nécessaires à l'expression spécifique dans les cellules sécrétrices des trichomes. L'élimination de ces motifs entraîne une diminution significative de l'expression ou une perte de la spécificité de l'expression. Ces motifs, au nombre de trois, sont définis par les séquences consensus suivantes, où les lettres K, W et Y représentent des bases d'acides nucléiques suivant le code de la nomenclature internationale avec K = G ou T, W = A ou T, et Y = C ou T :
- Motif 1 : 5'-KKTCGTWGCAWT -3' (SEQ ID NO : 6)
- Motif 2 : 5'-AGTAATWTYW-3' (SEQ ID NO : 7)
- Motif 3 : 5'- TTGTAGCWAW-3' (SEQ ID NO : 8)

Dans un mode de réalisation préféré, un fragment au sens de l'invention comprend au moins une séquence choisie parmi SEQ ID NO: 6, 7 ou 8. De même, un objet particulier de l'invention concerne tout acide nucléique comprenant une séquence choisie parmi SEQ ID NO: 6, 7 ou 8 ou un variant fonctionnel de celles-ci. De tels acides nucléiques sont typiquement des acides nucléiques recombinants ayant une activité de promoteur transcriptionnel. Il peut s'agir notamment de promoteurs chimériques, c'est-à-dire comprenant une séquence choisie parmi SEQ ID NO: 6, 7 ou 8 ou un variant fonctionnel de celles-ci, liée de manière opérationnelle à un promoteur minimum.

Le terme "variant fonctionnel" désigne tout acide nucléique présentant une ou plusieurs modifications (c'est-à-dire par exemple une mutation, délétion, ou addition d'une ou plusieurs bases) par rapport aux séquences parentes décrites dans la présente demande, et conservant une activité, soit de promoteur transcriptionnel, soit de signal spécificité d'expression, soit de transit. Les variants fonctionnels peuvent correspondre par exemple aux promoteurs issus des gènes correspondants dans d'autres espèces végétales. Ainsi, les travaux décrits dans les exemples de la présente demande ont porté sur des séquences de l'espèce *Nicotiana sylvestris,* un des parents diploïdes présumés du tabac cultivé, *N. tabacum.* Il est connu que les séquences de *N. sylvestris* et *N. tabacum* sont extrêmement proches (identité parfois de l'ordre de 99% entre les séquences de *N*. *tabacum* et *N. sylvestris),* de sorte que les promoteurs des gènes correspondants de *N. tabacum* constituent des variants fonctionnels au sens de l'invention, et peuvent être aisément préparés par des techniques classiques (hybridation, amplification, etc.). D'autres variants fonctionnels sont des acides nucléiques, synthétiques, recombinants ou naturels, dont la séquence hybride, en condition de stringence élevée, avec une des séquences SEQ ID NOS: 1, 2, 3, 4, 22 ou un fragment d'au moins 100 nucléotides consécutifs et qui possèdent une activité de promoteur transcriptionnel, notamment spécifique des trichomes glanduleux.

D'une manière générale, les variants fonctionnels sont des acides nucléiques présentant au moins 80, 85 ou 90% d'identité avec les séquences SEQ ID NOS: 1, 2, 3, 4 ou 22, ou avec leurs fragments d'au moins 100 nucléotides consécutifs, encore plus préférentiellement, plus de 95% d'identité obtenus par le programme d'alignement de séquences blastN (Altschul et al., 1990).

Le pourcentage d'identité est déterminé en comparant deux séquences dont un alignement optimal a été réalisé. Le pourcentage d'identité est calculé en déterminant le nombre de positions pour lesquelles un résidu identique apparaît sur les deux séquences à la même position divisé par le nombre de positions totales et multiplié par 100. L'alignement optimal des deux séquences peut être obtenu par exemple avec un algorithme de recherche d'homologie local (Smith & Watherman, 1981) ou des systèmes équivalents connus de l'homme du métier,

Les gènes identifiés ont également permis la caractérisation d'un peptide de transit, capable de cibler ou diriger une protéine hétérologue dans le compartiment chloroplastique des cellules végétales, notamment des trichomes. Ainsi, il est connu que les diterpène synthases possèdent, à leur extrémité N terminale, une séquence signal peptidique nécessaire à leur localisation dans le compartiment chloroplastique, lieu de biosynthèse du géranylgéarnylpyrophosphate (GGPP) (Trapp et Croteau, 2001). En utilisant le programme ChloroP (Emanuelsson *et al.,* 2000, Nielsen *et al.,* 1997), un peptide de transit putatif de 51 acides aminés (AA) a été identifié à partir de la séquence codante du gène TPS-02a. Ce peptide de transit (plus les 6 AA suivants) à été introduit dans la cassette N°1, en amont du gène *uidA* et en aval du promoteur TPS-02b. De cette façon, une fusion traductionnelle du peptide de transit avec la protéine GUS a été réalisée. Cette construction (cassette N°2) a été introduite dans un vecteur T-DNA (pBI121) possédant un gène de résistance pour la kanamycine sous la dépendance du promoteur du virus de la mosaïque du choux fleur (CaMV 35S). Ce T-DNA a été introduit dans *N. sylvestris* par transformation génétique en utilisant la souche *d'Agrobacterium tumefaciens* LBA4404 (Hoekema *et al.,* 1993).

Comme pour la cassette N°1, l'expression du gène *uidA* chez plusieurs transformants a été uniquement détectée dans les cellules sécrétrices des trichomes glanduleux. De plus, sous microscope, il a été mis en évidence que l'activité GUS était localisée dans les plastes, indiquant que la protéine GUS a été correctement ciblée dans le compartiment chloroplastique. Ces résultats ont démontré que la séquence étudiée, correspondant au peptide transit de la CBT-ol cyclase de *N. sylvestris* (TPS-02b), permet de diriger la localisation d'une protéine hétérologue dans le compartiment chloroplastique.

La présente demande décrit un peptide de transit dans le chloroplaste de cellules végétales, comprenant la séquence en acides aminés suivante MSQSISPLMFSHFAKFQSNIWRCNTSQLRVIHSSYASFGGRRKERVRRMNRAM DLSS (SEQ ID NO : 10) ou tout fragment ou variant fonctionnel de celle-ci, ainsi que dans un acide nucléique codant un peptide de transit dans le chloroplaste de cellules végétales, comprenant la séquence nucléotidique : « ATGAGTCAATCAATTTCTCCATTAATGTTTTCTCACTTTGCAAAATTTCAGT CGAATATTTGGAGATGCAATACTTCTCAACTCAGAGTTATACACTCATCATA TGCCTCTTTTGGAGGGAGAAGAAAAGAGAGAGTAAGAAGAATGAATCGAG CAATGGATCTTTCTTCA » (SEQ ID NO : 9), ou tout fragment variant fonctionnel de celle-ci.

L'invention concerne également toute cassette d'expression recombinante, caractérisée en ce qu'elle comprend un gène et/ou un promoteur d'intérêt lié(s) de manière opérationnelle à un acide nucléique tel que défini ci-dessus.
Le terme cassette d'expression désigne une construction d'acide nucléique comprenant une région codante et une région régulatrice, liées de manière opérationnelle. L'expression "lié de manière opérationnelle" indique que les éléments sont combinés de manière à ce que l'expression de la séquence codante (le gène d'intérêt) et/ou le ciblage de la protéine codée soient sous contrôle du promoteur transcriptionnel et/ou du peptide de transit. Typiquement, la séquence du promoteur est placée en amont du gène d'intérêt, à une distance de celui-ci compatible avec le contrôle de l'expression. De même, la séquence du peptide de transit est généralement fusionnée en amont de la séquence du gène d'intérêt, et en phase avec celui-ci, et en aval de tout promoteur. Des séquences d'espacement peuvent être présentes, entre les éléments régulateurs et le gène, dès lors qu'elles n'empêchent pas l'expression et/ou le ciblage.

Le gène d'intérêt peut être tout acide nucléique (par exemple un ADN ou un ARN) comportant une région codant un produit d'expression (par exemple un ARNm ou une protéine). Dans un mode de mise en oeuvre préféré, le gène d'intérêt code une protéine thérapeutique ou phytosanitaire, une enzyme, une protéine de résistance, un activateur de transcription ou un génome viral.

Un autre objet de l'invention concerne tout vecteur (d'expression) comprenant un acide nucléique ou une cassette tels que définis précédemment. Le vecteur peut être un ADN ou un ARN, circulaire ou non, simple- ou double-brin. Il s'agit typiquement d'un plasmide, phage, virus, cosmide, chromosome artificiel, etc. Il s'agit avantageusement d'un vecteur de plante, c'est-à-dire capable de transformer une cellule végétale. Des exemples de vecteurs de plantes sont décrits dans la littérature, parmi lesquels on peut citer notamment les plasmides T-DNA de *A. tumefaciens* pBIN19 *(Bevan, 1984),* pPZP100 *(Hajdukewicz et al., 1994),* série pCAMBIA (R. *Jefferson, CAMBIA, Australie), ,.* Les vecteurs de l'invention peuvent comprendre, en outre, une origine de réplication et/ou un gène de sélection et/ou une séquence de recombinaison végétale, etc. Les vecteurs peuvent être construits par des techniques classiques de biologie moléculaire, bien connues de l'homme du métier, utilisant par exemple des enzymes de restriction, de ligature, des clonages, réplication, etc. Des exemples spécifiques de vecteurs au sens de l'invention sont fournis dans la partie expérimentale, et incluent notamment pLIBRO-01 et pLIBRO-02.

Les constructions génétiques de l'invention peuvent être utilisées pour la modification génétique de plantes, et notamment pour l'introduction et l'expression de protéines dans des tissus végétaux, y compris des plantes entières.

L'introduction des constructions de l'invention dans une cellule ou un tissu végétal, y compris une graine ou plante, peut être réalisée par toute technique connue de l'homme du métier. Les techniques de transgénèse végétale sont bien connues dans le domaine, et comprennent par exemple l'utilisation de la bactérie *Agrobacterium tumefaciens,* l'électroporation, le transfert conjugatif, des techniques biolistiques, etc.

Une technique communément utilisée repose sur l'utilisation de la bactérie *Agrobacterium tumefaciens,* qui consiste essentiellement à introduire la construction d'intérêt (acide nucléique, cassette, vecteur, etc.) dans la bactérie *A. tumefaciens,* puis à mettre en contact cette bactérie transformée avec des disques de feuilles de la plante choisie. L'introduction de la cassette d'expression dans la bactérie est typiquement réalisée en utilisant comme vecteur le plasmide Ti (ou T-DNA), qui peut être transféré dans la bactérie par exemple par choc thermique. L'incubation de la bactérie transformée avec les disques foliaires permet d'obtenir le transfert du plasmide Ti dans le génome des cellules des disques. Ceux-ci peuvent éventuellement être cultivés dans des conditions appropriées pour reconstituer une plante transgénique dont les cellules comprennent la construction de l'invention. Pour plus de détails ou des variantes de mise en oeuvre de la technique de transformation par *A. tumefaciens,* on peut se référer par exemple à Horsch et al.,1985 ou Hooykaas and Schilperoort, 1992.

Une autre technique de transformation végétale est basée sur la projection de microparticules (typiquement des microbilles) auxquelles sont attachées les constructions génétiques, directement sur des cellules végétales, suivie de la culture de ces cellules pour reconstituer une plante transgénique. Les particules utilisées sont typiquement des particules d'or, qui sont projetées typiquement au moyen d'un canon à particules (voir notamment Russell et al., In Vitro Cell. Dev. Biol., 1992, 28P, p. 97-105).

La technique de microinjection repose essentiellement sur l'injection des constructions génétiques dans des embryons ou protoplastes végétaux, puis à cultiver ces tissus de manière à régénérer des plantes complètes. D'autres techniques de transgénèse végétale sont bien connues, ou d'autres protocoles mettant en oeuvre les techniques ci-dessus sont décrits dans l'art antérieur (Siemens, J and Schieder, 1996) et peuvent être appliqués à la présente invention.

Une fois régénérées, les plantes transgéniques peuvent être testées pour l'expression du produit d'intérêt dans les trichomes. Ceci peut être réalisé en récoltant l'exsudat des feuilles et en testant la présence du produit dans cet exsudat, lorsque le produit est destiné à être sécrété. Ceci peut également être fait en analysant la présence de produit d'expression du gène d'intérêt dans les feuilles et, plus particulièrement, dans les cellules de trichome (par exemple en analysant les ARNm ou l'ADN génomique au moyen de sondes ou d'amorces spécifiques). Les plantes peuvent éventuellement être sélectionnées, croisées, traitées, etc. pour obtenir des plantes présentant des niveaux d'expression améliorés.

A cet égard, un autre objet de l'invention réside dans une cellule modifiée comprenant une cassette ou un vecteur tels que définis précédemment. Il peut s'agir par exemple d'une cellule de plante, notamment de la famille des Solanacées, Astéracées, Cannabacées ou Lamiacées. Les cellules peuvent être cultivées *in vitro,* et utilisées pour reconstituer des tissus ou plantes entières, pour produire des protéines en culture, ou encore pour étudier les propriétés de gènes ou protéines d'intérêt (par exemple en génomique fonctionnelle).

Un autre objet de l'invention réside également dans une plante ou graine comprenant une cassette d'expression ou un vecteur tels que définis précédemment.

L'invention concerne encore un procédé de production d'une protéine dans une plante, notamment dans le trichome glanduleux d'une plante, comprenant l'introduction dans ladite plante d'une cassette ou d'un vecteur tels que définis précédemment, comprenant un gène codant ladite protéine.

Un autre aspect de l'invention concerne un procédé de production d'une protéine dans une plante, notamment dans le trichome glanduleux d'une plante, comprenant l'introduction dans une cellule ou graine de plante d'une cassette ou d'un vecteur tels que définis précédemment, comprenant un gène codant ladite protéine, et la reconstitution d'une plante à partir de ladite cellule ou graine.

L'invention concerne encore tout procédé de production d'une plante exprimant une protéine recombinante, comprenant l'introduction dans une cellule ou graine de plante d'une cassette ou d'un vecteur tels que définis précédemment, comprenant un gène codant ladite protéine, et la reconstitution d'une plante à partir de ladite cellule ou graine.

Avantageusement, la protéine recombinante exprimée dans le trichome conduit à la production par les cellules sécrétrices d'une molécule sécrétée dans l'exsudat de la surface des feuilles et/ou à une modification de la composition de l'exsudat.

Avantageusement, la plante sécrète la protéine dans le trichome glanduleux, et la protéine est récupérée à partir de l'exsudat de la surface des feuilles.

L'invention peut ainsi être utilisée pour modifier des plantes, cellules ou tissus végétaux, pour leur faire exprimer différents produits d'intérêt.

Elle peut notamment être utilisée pour l'expression de produits d'intérêt spécifiquement dans les cellules sécrétrices de trichomes glanduleux des plantes supérieures (notamment les Angiospermes). L'invention est applicable notamment à toutes les plantes de familles possédant des trichomes glanduleux, par exemple les Astéracées, Solanacées, Cannabacées et Lamiacées. Elle est particulièrement adaptée aux plantes de la famille des Solanacées, telles que par exemple des genres Solanum, Lycopersicon, Capsicum, Petunia, Datura, Atropa, etc., et aux Nicotianées, par exemple *Nicotiana sylvestris* et *N. tabacum.*

Le produit d'intérêt peut être toute protéine recombinante, y compris des peptides, enzymes, anticorps, etc. Il peut s'agir notamment d'une protéine présentant une activité biologique d'intérêt industriel, par exemple médical ou phytosanitaire. Il peut également s'agir d'une protéine destinée à conférer à la plante des propriétés particulières (notamment une modification de la composition de la sécrétion (exsudat)), comme la résistance à des pathogènes (insectes ravageurs, champignons, bactéries, virus, etc.), une meilleure croissance, une teneur modifiée en métabolite ou une voie de synthèse modifiée, etc.

L'invention peut également être utilisée pour exprimer, dans une cellule végétale ou une plante, un activateur de la transcription. Dans ce cas, l'activateur transcriptionnel produit permettra de contrôler l'expression d'une protéine d'intérêt placée sous contrôle d'un promoteur répondant à cet activateur. Un tel système binaire, utilisant deux cassettes (présentes sur un même vecteur ou sur des vecteurs distincts), permet d'amplifier l'expression spécifique obtenue grâce à l'invention. On peut citer à titre d'exemple un système comprenant une première cassette avec le gène codant pour un activateur de transcription (exemple GAL4) sous le contrôle du promoteur trichome spécifique, et une autre cassette comprenant le gène codant pour la protéine d'intérêt sous le contrôle d'éléments régulateurs reconnus par le facteur de transcription (Gal4).

Un autre de système d'amplification consiste à utiliser un vecteur viral à ARN. Certains virus de plantes à ARN codent en effet pour une ARN-polymérase ARN-dépendante qui permet l'amplification de transcrits d'un gène donné. Dans ce mode de réalisation, le gène d'intérêt est cloné en aval du promoteur de cette ARN polymérase, en lieu et place de la phase de lecture codant pour la protéine du manteau ("*coat protein")* du virus. Le virus est lui-même placé sous le contrôle du promoteur spécifique. L'expression du virus est donc restreinte aux cellules sécrétrices des trichomes, permettant ainsi une amplification sélective de l'expression du gène dans ces cellules.

D'autres aspect et avantages de la présente invention apparaîtront à la lecture des exemples qui suivent, qui doivent être considérés comme illustratifs et non limitatifs.

### LEGENDE DES FIGURES

Figure 1: Alignement des séquences de promoteurs de invention : Les séquences des 4 promoteurs TPS d'une longueur d'environ 1 kb ont été alignés avec le programme d'alignement AlignX (VectorNTI suite, InformaxInc®). Les motifs nécessaires à l'activité trichome spécifique sont encadrés en gris.
Figure 2: Visualisation sous microscope (A et B) et loupe binoculaire (B et D) des cellules glandulaires des trichomes de différents organes après coloration histochimique des tissues avec le substrat X-Gluc. L'expression du gène rapporteur *uidA,* visualisée par une coloration bleue, est uniquement détectée dans les cellules glandulaires du trichome. Les cellules de la base du trichome ne montrent aucune coloration. Les colorations ont été réalisées sur deux lignées de tabac transformées avec la construction pLIBRO-01. A et C : trichomes de feuilles, B : trichomes de tiges, D : trichomes de sépales.

### EXEMPLES

### 1. Isolement et caractérisation des séquences

L'ADN génomique a été extrait à partir de feuilles de tabac *(N. sylvestris* ou *N. tabacum)* à l'aide d'un kit commercial de Qiagen® (DNeasy Plant Maxi Kit). Le clonage des régions promotrices des différents gènes TPS (TPS02a, 02b, 03, et 04) a été réalisé par la technique de « adaptater-anchor PCR (Siebert et al., 1995) ». Brièvement l'ADN génomique a été digéré séparément par différentes enzymes de restriction générant des bouts francs (DraI, SspI, NaeI....) et ligaturé à un adaptateur composé des amorces suivantes :
ADPR1 5'-CTAATACGACTCACTATAGGGCTCGAGCGGCCGCCCGGGGAGGT (SEQ ID No 14) et ADPR2 5'-ACCTCCCC (SEQ ID No 15). L'identification de la région promotrice du gène TPS02 a ensuite été obtenue en réalisant successivement deux réactions de PCR (PCR1 puis PCR2) avec les primers complémentaires de l'adaptateur (AP1 & AP2) et différentes amorces de la séquence codante du gène CYC-2 publié (N° Genbank AF401234, NID : AY495694). Pour identifier les séquences promotrices d'environ 1 kb, la réaction de PCR1 a été réalisée avec l'amorce AP1 (5'-GGATCCTAATACGACTCACTATAGGGC, SEQ ID No 16) et l'amorce externe 2A04 (5'-AGAGGCATATGATGAGTGTATAACTC, SEQ ID No 17). Une fraction de la réaction de PCR1 a été utilisée pour réaliser la réaction de PCR2 avec l'amorce AP2 (5'-CTATAGGGCTCGAGCGGC, SEQ ID No 18) et l'amorce interne 2A02 (5'-ACCTCCAAATATTCGACTGAAATTT, SEQ ID No 19). Les fragments obtenus par cette méthode ont une taille qui varie entre 200 et 1000 pb. Afin d'isoler des séquences plus importantes, le même processus a été répété à partir des séquences nouvellement identifiées. Par exemple, pour identifier la séquence promotrice de 1,7 kb correspondante au gène TPS02b, la réaction de PCR1 a été réalisée sur le même matériel génomique et dans les mêmes conditions PCR mais en utilisant l'amorce externe 2G07 (5'-GTCATGTGCTTGAAGTTACCATATC, SEQ ID No 20) et l'amorce interne 2G08 (5'-GTTACCATATCAATATTATTTACCAT, SEQ ID No 21). Les amplifications PCR ont toutes été réalisées dans les mêmes conditions standard de PCR avec 0.25 unité de l'enzyme Taq (fournisseur Eppendorf) dans 25µl de mélange de réaction dans les conditions suivantes : 1 cycle à 94°C pendant 2 min, puis 35 cycles à 94°C 30 sec, 68°C 30 sec et 1 cycle à 72°C 5 min en utilisant 50 ng d'ADN génomique. Pour le clonage du promoteur TPS-02b (PromTPS02b) dans le vecteur binaire, une nouvelle amplification par PCR a été réalisée à partir d'ADN génomique avec l'amorce sens, 3A04 (séquence 5'-CT**AAGCTT**AATTTATTTTTGTAAAACTTC, SEQ ID NO: 11) qui possède un site HindIII en 5' et l'amorce antisens, 3A03 (séquence 5'-AT**GGATCC**CTCTTTCTCTCGCCAAACGAGT, SEQ ID NO: 12) qui possède un site BamH1 en 5'. Pour le clonage du promoteur TPS-02b comprenant le peptide transit (promTPS02b-TP), l'amorce sens 3A04 a été utilisée avec l'amorce antisens 4C01 (5'-T**GGATCCT**GAAGAAAGATCCATTGCTCGA, SEQ ID NO: 13), comprenant un site HindIII en 5'. Pour le clonage du promoteur TPS-02b_1.7kb (PromTPS02b_1.7, SEQ ID No 22), l'amorce sens 4E07 qui possède un site SacI en 5' (séquence 5' T**GAGCTC**AAAGAGGTGAAACCTAATCTAGTATGCAA 3', SEQ ID No 23) a été utilisé avec l'amorce 3A03. Les amplifications ont été réalisée avec de l'enzyme Taq (Eppendorf®) dans les conditions suivantes : 1 cycle à 94°C pendant 2 min, et 35 cycles à 94°C 30 sec, 57°C 30 sec, 72°C 1 min et 1 cycle à 72°C 5 min en utilisant 50 ng d'ADN génomique pour une réaction de 25 µl. Les produits PCR ont été purifiés sur une colonne Qiaquick (Qiagen®) et clonés dans le vecteur pGEM-T (Promega®). Les plasmides ainsi obtenus ont été nommés respectivement pPromTPS02b et pPromTPS02b-TP. Les clones ont été séquencés, et la séquences des régions promotrices de 1 kb obtenues est représentée dans SEQ ID NO: 1-4. Les séquences ont été alignées (Figure 1), faisant apparaître une identité de 93% ou plus. Un alignement avec les séquences de 1,7 kb donne des pourcentages d'identité supérieurs à 90%.

### 2. Construction des cassettes et vecteurs de transformation

Le promoteur CaMV 35S situé en amont du gène *uidA* du vecteur binaire pBI121 (AF485783) a été éliminé par digestion enzymatique avec les enzymes HindIII et BamH1 et purifié sur gel d'agarose sur une colonne Qiaquick (Qiagen®). Les séquences promotrices PromTPS02b et PromTPS02b-TP ont été purifiées sur une colonne Qiaquick (Qiagen®) après digestion des plasmides pPromTPS02b et pPromTPS02b-TP par les enzymes HindIII et BamH1. Les séquences promotrices ont été introduites dans le vecteur pBI121 au niveau des sites HindIII et BamHI en remplacement du promoteur CaMV 35S générant ainsi une fusion transcriptionelle avec le gène *uidA.* Les constructions sont nommées pLIBRO-18 et pLIBRO-19 respectivement. Les deux vecteurs binaires pLIBRO-01 et 02 ont été introduits dans la souche d'Agrobacterium LBA4404 par électroporation.
Le promoteur promTPS02b_1.7 a été cloné dans le site EcoRI du vecteur pCAMBIA1391Z (NID : AF234312) donnant ainsi le vecteur pLIBRO-32.

### 3. Transformation génétique

Des lignées de tabacs transgéniques *(N. sylvestris)* portant les constructions pLIBRO-01, pLIBRO-02 et pLIBRO-32 ont été obtenues par transformation génétique avec les souches *d'Agrobacterium* correspondantes en utilisant la méthode des disques foliaires (Horsch et al., 1985). La sélection des lignées transformées a été réalisée avec 100 mg/L de sulfate de kanamycine et l'élimination de la souche d'Agrobacterium avec 250 mg/L de Carbenicilline.

### 4. Analyse de l'expression du gène uidA

La visualisation de l'expression du gène uidA a été réalisée par un test histochimique GUS selon le protocole décrit par Jefferson *et al.,* (1987). La visualisation de la coloration obtenue a été réalisée sous une loupe binoculaire.

### 5. Mise en évidence d'une expression spécifique des trichomes

L'expression du gène *uidA* dans plusieurs lignées de tabac transgéniques *(N. sylvestris)* portant la construction pLIBRO-01 et pLIBRO-32 a été déterminée. Les résultats obtenus pour la construction pLIBRO-01 sont présentés sur la Figure 2 (voir légende plus haut).

Ces résultats montrent une expression dans les cellules sécrétrices, mais pas dans les cellules constituant le pied des trichomes. En outre, aucune expression du gène *uidA* n'a été trouvée dans les autres organes de la plante. Ces résultats démontrent que la séquence étudiée permet de diriger l'expression d'un gène hétérologue dans les cellules sécrétrices des trichomes glanduleux de manière très spécifique. Des résultats identiques ont été obtenus avec la construction pLIBRO-32 (résultats non présentés).

### 6. Mise en évidence d'un ciblage dans le chloroplaste

L'expression du gène *uidA* dans plusieurs lignées de tabac transgéniques *(N. sylvestris)* portant la construction pLIBRO-02 a été déterminée.

Comme pour la cassette N°1, l'expression du gène *uidA* chez plusieurs transformants a été uniquement détectée dans les cellules sécrétrices des trichomes glanduleux. De plus, sous microscope, il a été mis en évidence que l'activité GUS était localisée dans les cellules chloroplastiques, indiquant que la protéine GUS a été correctement ciblée dans le compartiment chloroplastique. Ces résultats ont démontré que la séquence du peptide de transit de l'invention permet de diriger la localisation d'une protéine hétérologue dans le compartiment chloroplastique.

**Tableau 1**

| Gène Abréviation | Nom du gène | Plantes | Promoteur (bp) | Plante transformée | Expression | Références |
|---|---|---|---|---|---|---|
| LTP3 | Lipid transfer protein | Cotton | 1548 | Tabac | Trichomes, épiderme périphérique des feuilles et tissus vasculaires | Liu et al., 2000, BBA, 1487:106111 |
| | | | 1143 | | | |
| | | | 614 | | | |
| LTP6 | Lipid transfer protein | Cotton | 447 | Tabac | trichomes et cellules de guarde Hsu des stomates | et al., 1999, Plant science, 143:6370 |
| | | | 272 | | | |
| wax9D | Lipid transfer protein | Brassica oleracca | 972 | Tabac | Epidermes de feuilles, tiges et fleurs, pétales, sépales, ovules, et trichomes | Pyee and Kolattukudy, 1995, Plant J. 7:4559 |
| LTP1 | Lipid transfer protein | Arabidopsis | 1149 | Arabidopsis | Cellules épidermiques de tissues divers | Thoma et al., 1994, Plant Physiol. 105 3545 |
| CYC71D16 | CBTol hydroxylase | Tobacco | 1852 | Tabac | Trichomes | Wang et al., 2002, J.ofExp. Bot. 18911897 |

**Tableau 2**

| | NsTPS02a-Prom1kb | NsTPS02b-Prom1kb | NsTPS03-Prom1kb | NsTPS04-Prom1Kb |
|---|---|---|---|---|
| NsTPS02a-Prom1kb | 100 | 96 | 93 | 94 |
| NsTPS02b-Prom1kb | | 100 | 93 | 93 |
| NsTPS03-Prom1kb | | | 100 | 93 |
| NsTPS04-PromlKb | | | | 100 |

### REFERENCES

Altschul SF, Gish W, Miller W, Myers EW, Lipman, DJ (1990) J. Mol. Biol. 215:403-410
Bevan M (1984) Nuc. Acid. Res. 12 : 8711-8721.
Bohlmann J, Meyer-Gauen G, Croteau R (1998) Proc. Natl. Acad. Sci. U.S.A. 95:4126-4133.
Emanuelsson O, Nielsen H, Brunak S and Von Heijne G (2000) J. Mol. Biol. 300, 1005-1016
Jefferson RA. (1987) Plant Mol. Biol. Rep. 5:387-405
Hajdukiewicz P, Svab Z, Maliga P (1994) Plant Mol. Biol. 25: 989-994.
Nielsen H, Engelbrecht J, Brunak S, and Von Heijne G (1997) Prot. Eng. 10: 1-6 Hoekema A, Hirsch PR, Hooykaas PJJ and Schilperoort RA (1983) Nature 303:179-180 Hooykaas PJJ and Schilperoort RA (1992) Plant Mol. Biol. 19:15-38 Horsch RB, Fry J, Hoffmann NL, Wallroth M, Eichholtz D, Rogers SG, and Fraley RT (1985) Science 227:1229-1231
Rohmer M, Seeman M, Horbach S, Bringer-Meryer S, and Sahm H (1996) J. Am. Chem. Soc. 118:2564-2566
Siebert PD, Chenchick A, Kellogg DE, Lukyanov KA, Lukyanov SA (1995) Nucleic Acids Res. 23 : 1087-1088
Siemens J, and Schieder A (1996) Plant Tiss. Cult. Biotechnol. 2:66-75
Smith TF, and Watherman MS (1981) Add. Appl. Math. 2 :482
Trapp SC, and Croteau RB (2001) Genetics 158: 811-832
Wagner GJ, Gan S, Wang E, Wang R., 2003 BREVET US 2003/0100050 A1
Wagner GJ, Wang E, Shepherd RW (2004) Annals of Botany 93:3-11
Wang E, Wang R, DeParasis J, Loughrin J, Gan S, and Wagner GJ (2001) Nature 19:371-374
Wang E, Gan S, and Wagner GJ (2002) J. Exp. 53:1891-1897
Wang E and Wagner GJ (2003) Planta 216:686-691

## Revendications

1. Acide nucléique ayant une activité de promoteur transcriptionnel spécifique des cellules sécrétrices dans les trichomes glanduleux, **caractérisé en ce qu'**il comprend une séquence sélectionnée parmi la SEQ ID NO: 2, un fragment de celle-ci d'au moins 100 nucléotides consécutifs ou un variant fonctionnel de celle-ci présentant au moins 80 % d'identité avec celle-ci ou ledit fragment.

2. Acide nucléique selon la revendication 1, **caractérisé en ce qu'**il comprend une séquence présentant au moins 90% d'identité avec la SEQ ID No: 2, ou un fragment de celle-ci d'au moins 100 nucléotides consécutifs,.

3. Acide nucléique selon la revendication 1 ou 2, **caractérisé en ce qu'**il comprend une séquence sélectionnée parmi les SEQ ID Nos 1, 3, 4 et 22, ou un fragment de celle-ci d'au moins 100 nucléotides consécutifs.

4. Acide nucléique selon l'une quelconque des revendications 1 à 3, **caractérisé en ce qu'**il comprend une séquence choisie parmi SEQ ID NO : 6, 7 et 8.

5. Cassette d'expression recombinante, **caractérisée en ce qu'**elle comprend un gène d'intérêt lié de manière opérationnelle à un acide nucléique selon l'une des revendications 1 à 4.

6. Cassette d'expression selon la revendication 5, **caractérisée** an ce que le gène d'intérêt code une enzyme conduisant à la modification de la composition de l'exsudat sécrété par les trichomes.

7. Cassette d'expression selon la revendication 5 ou 6, **caractérisée en ce que** le gène d'intérêt code une protéine thérapeutique ou phytosanitaire, une enzyme, une protéine de résistance, un activateur de transcription ou un génome viral.

8. Cassette d'expression selon l'une quelconque des revendications 5 à 7, **caractérisée en ce qu'**elle comprend en outre un acide nucléique codant un peptide de transit dans le chloroplaste de cellules végétales, comprenant la séquence MSQSISPLMFSHFAKFQSNIWRCNTSQLRVIHSSYASFGGRRKERVRRMNRAMDLSS (SEQ ID NO : 10).

9. Vecteur d'expression comprenant un acide nucléique selon l'une des revendications 1 à 4 ou une cassette selon l'une des revendications 5 à 8.

10. Cellule modifiée comprenant une cassette selon l'une des revendications 5 à 8 ou un vecteur selon la revendication 9.

11. Cellule selon la revendication 10, **caractérisée en ce qu'**il s'agit d'une cellule de plante, notamment de la famille des Solanacées, Astéracées, Cannabacées ou Lamiacées.

12. Procédé de production d'une protéine dans une plante, comprenant l'introduction dans ladite plante d'une cassette selon l'une des revendications 5 à 8 ou d'un vecteur selon la revendication 9, comprenant un gène codant ladite protéine.

13. Procédé de production d'une protéine dans une plante, comprenant l'introduction dans une cellule ou graine de plante d'une cassette selon l'une des revendications 5 à 8 ou d'un vecteur selon la revendication 9, comprenant un gène codant ladite protéine, et la reconstitution d'une plante à partir de ladite cellule ou graine.

14. Procédé de production d'une plante exprimant une protéine recombinante, comprenant l'introduction dans une cellule ou graine de plante d'une cassette selon l'une des revendications 5 à 8 ou d'un vecteur selon la revendication 9, comprenant un gène codant ladite protéine, et la reconstitution d'une plante à partir de ladite cellule ou graine.

15. Procédé selon l'une quelconque des revendications 12 à 14, **caractérisé en ce que** la plante sécrète la protéine dans le trichome glanduleux, et **en ce que** la protéine est récupérée à partir de l'exsudat de la surface des feuilles.

16. Procédé selon l'une quelconque des revendications 12 à 15, **caractérisé en ce que** la plante est une plante de la famille des Solanacées, Asteracées, Cannabacées ou Lamiacées.

17. Plante ou graine comprenant une cassette d'expression selon l'une des revendications 5 à 8 ou un vecteur selon la revendication 9.

18. Utilisation d'un acide nucléique selon l'une des revendications 1 à 4 pour l'expression d'une protéine spécifiquement dans les trichomes glanduleux d'une plante.

## Claims

1. A nucleic acid having transcriptional promoter activity specific of secretory cells in glandular trichomes, **characterized in that** it comprises a sequence selected in the group consisting of SEQ ID No. 2, a fragment thereof having at least 100 consecutive nucleotides or a functional variant thereof displaying at least 80 % identity with said sequence or said fragment.

2. The nucleic acid according to claim 1, **characterized in that** it comprises a sequence displaying at least 90 % identity with SEQ ID No 2, or a fragment thereof having at least 100 consecutive nucleotides.

3. The nucleic acid according to claim 1 or 2, **characterized in that** it comprises a sequence selected in the group consisting of SEQ ID Nos. 1, 3, 4 and 22 or a fragment thereof having at least 100 consecutive nucleotides.

4. The nucleic acid according to any one of claims 1 to 3, **characterized in that** it comprises a sequence selected in the group consisting of SEQ ID Nos. 6, 7 and 8.

5. A recombinant expression cassette, **characterized in that** it comprises a gene of interest operatively linked to a nucleic acid according to any one of claims 1 to 4.

6. The expression cassette according to claim 5, **characterized in that** the gene of interest codes for an enzyme leading to the modification of the composition of the exudate secreted by the trichomes.

7. The expression cassette according to claim 5 or 6, **characterized in that** the gene of interest codes for a therapeutic or phytosanitary protein, an enzyme, a resistance protein, a transcriptional activator or a viral genome.

8. The expression cassette according to any one of claims 5 to 7, **characterized in that** it further comprises a nucleic acid coding for a plant cell chloroplast transit peptide, comprising the sequence MSQSISPLMFSHFAKFQSNIWRCNTSQLRVIHSSYASFGGRRKERVRRMNRAMDLSS (SEQ ID No. 10).

9. An expression vector comprising a nucleic acid according to any one of claims 1 to 4 or a cassette according to any one of claims 5 to 8.

10. A recombinant cell comprising a cassette according to any one of claims 5 to 8 or a vector according to claim 9.

11. The cell according to claim 10, **characterized in that** it is a plant cell, in particular from the family of Solanaceae, Asteraceae, Cannabaceae or Lamiaceae.

12. A method for producing a protein in a plant, comprising introducing into said plant a cassette according to any one of claims 5 to 8 or a vector according to claim 9, comprising a gene coding for said protein.

13. A method for producing a protein in a plant, comprising introducing into a plant cell or seed a cassette according to any one of claims 5 to 8 or a vector according to claim 9, comprising a gene coding for said protein, and regenerating a plant from said cell or seed.

14. A method for producing a plant expressing a recombinant protein, comprising introducing into a plant cell or seed a cassette according to any one of claims 5 to 8 or a vector according to claim 9, comprising a gene coding for said protein, and regenerating a plant from said cell or seed.

15. The method according to any one of claims 12 to 14, **characterized in that** the plant secretes the protein in the glandular trichome, and **in that** the protein is recovered from the exudate at the leaf surface.

16. The method according to any one of claims 12 to 15, **characterized in that** the plant is a plant from the family of Solanaceae, Asteraceae, Cannabaceae or Lamiaceae.

17. A plant or seed comprising an expression cassette according to any one of claims 5 to 8 or a vector according to claim 9.

18. Use of a nucleic acid according to any one of claims 1 to 4 for expressing a protein specifically in the glandular trichomes of a plant.

## Patentansprüche

1. Nukleinsäure mit Transkriptionspromotor-Aktivität, die für sekretorische Zellen in den glandulären Trichomen spezifisch ist, **dadurch gekennzeichnet, dass** sie eine Sequenz, ausgewählt aus SEQ ID NR: 2, ein Fragment davon mit wenigstens 100 aufeinanderfolgenden Nukleotiden oder eine funktionelle Variante davon umfasst, die eine Identität von wenigstens 80% mit dieser Sequenz oder besagtem Fragment aufweist.

2. Nukleinsäure gemäß Anspruch 1, **dadurch gekennzeichnet, dass** sie eine Sequenz, die eine Identität von wenigstens 90% mit der SEQ ID NR: 2 aufweist, oder ein Fragment davon mit wenigstens 100 aufeinanderfolgenden Nukleotiden umfasst.

3. Nukleinsäure gemäß Anspruch 1 oder 2, **dadurch gekennzeichnet, dass** sie eine Sequenz, ausgewählt aus SEQ ID NR: 1, 3, 4 und 22, oder ein Fragment davon mit wenigstens 100 aufeinanderfolgenden Nukleotiden umfasst.

4. Nukleinsäure gemäß einem der Ansprüche 1 bis 3, **dadurch gekennzeichnet, dass** sie eine Sequenz, ausgewählt aus SEQ ID NR: 6, 7 und 8, umfasst.

5. Rekombinante Expressionskassette, **dadurch gekennzeichnet, dass** sie ein Gen von Interesse umfasst, das operabel an eine Nukleinsäure gemäß einem der Ansprüche 1 bis 4 gebunden ist.

6. Expressionskassette gemäß Anspruch 5, **dadurch gekennzeichnet, dass** das Gen von Interesse ein Enzym codiert, das zur Modifikation der Zusammensetzung des von den Trichomen sezernierten Exsudates führt.

7. Expressionskassette gemäß Anspruch 5 oder 6, **dadurch gekennzeichnet, dass** das Gen von Interesse ein therapeutisches oder phytosanitäres Protein, ein Enzym, ein Resistenzprotein, einen Transkriptionsaktivator oder ein virales Genom codiert.

8. Expressionskassette gemäß einem der Ansprüche 5 bis 7, **dadurch gekennzeichnet, dass** sie außerdem eine Nukleinsäure umfasst, codierend ein Chloroplasten-Transitpeptid pflanzlicher Zellen, umfassend die Sequenz MSQSISPLMFSHFAKFQSNIWRCNTSQLRVIHSSYASFGGRRKERVRRMNRAMDLSS (SEQ ID NR: 10).

9. Expressionsvektor, umfassend eine Nukleinsäure gemäß einem der Ansprüche 1 bis 4 oder eine Kassette gemäß einem der Ansprüche 5 bis 8.

10. Veränderte Zelle, umfassend eine Kassette gemäß einem der Ansprüche 5 bis 8 oder einen Vektor gemäß Anspruch 9.

11. Zelle gemäß Anspruch 10, **dadurch gekennzeichnet, dass** es sich um eine Pflanzenzelle, insbesondere der Familie der Solanaceae, Asteraceae, Cannabaceae oder Lamiaceae, handelt.

12. Verfahren zur Herstellung eines Proteins in einer Pflanze, umfassend die Einführung in besagte Pflanze einer Kassette gemäß einem der Ansprüche 5 bis 8 oder eines Vektors gemäß Anspruch 9, umfassend ein besagtes Protein codierendes Gen.

13. Verfahren zur Herstellung eines Proteins in einer Pflanze, umfassend die Einführung in eine Zelle oder einen Samen der Pflanze einer Kassette gemäß einem der Ansprüche 5 bis 8 oder eines Vektors gemäß Anspruch 9, umfassend ein besagtes Protein codierendes Gen, und die Rekonstitution einer Pflanze aus besagter Zelle oder besagtem Samen.

14. Verfahren zur Herstellung einer ein rekombinantes Protein exprimierenden Pflanze, umfassend die Einführung in eine Zelle oder einen Samen der Pflanze einer Kassette gemäß einem der Ansprüche 5 bis 8 oder eines Vektors gemäß Anspruch 9, umfassend ein besagtes Protein codierendes Gen, und die Rekonstitution einer Pflanze aus besagter Zelle oder besagtem Samen.

15. Verfahren gemäß einem der Ansprüche 12 bis 14, **dadurch gekennzeichnet, dass** die Pflanze das Protein in das glanduläre Trichom sezerniert, und dass das Protein aus dem Exsudat der Blattoberfläche wiedergewonnen wird.

16. Verfahren gemäß einem der Ansprüche 12 bis 15, **dadurch gekennzeichnet, dass** die Pflanze eine Pflanze der Familie der Solanaceae, Asteraceae, Cannabaceae oder Lamiaceae ist.

17. Pflanze oder Samen, umfassend eine Expressionskassette gemäß einem der Ansprüche 5 bis 8 oder einen Vektor gemäß Anspruch 9.

18. Verwendung einer Nukleinsäure gemäß einem der Ansprüche 1 bis 4 zur Expression eines Proteins spezifischerweise in den glandulären Trichomen einer Pflanze.
